## Europäisches Patentamt

## European Patent Office

## Office européen des brevets

(11) Veröffentlichungsnummer : **0 191 851**
**B1**

(12) **EUROPÄISCHE PATENTSCHRIFT**

(45) Veröffentlichungstag der Patentschrift :
26.04.89

(21) Anmeldenummer : 85904442.2

(22) Anmeldetag : 20.08.85

(86) Internationale Anmeldenummer :
PCT/EP 85/00427

(87) Internationale Veröffentlichungsnummer :
WO/8601412 (13.03.86 Gazette 86/06)

(51) Int. Cl.⁴ : **A 61 M   1/00, A 61 M 27/00**

(54) UNTERDRUCKFLASCHE FÜR DIE REDON-WUNDDRAINAGE.

(30) Priorität : 29.08.84 CH 4138/84

(43) Veröffentlichungstag der Anmeldung :
27.08.86 Patentblatt 86/35

(45) Bekanntmachung des Hinweises auf die Patenterteilung : 26.04.89 Patentblatt 89/17

(84) Benannte Vertragsstaaten :
AT BE CH DE FR GB IT LI LU NL SE

(56) Entgegenhaltungen :
EP—A— 0 036 546
US—A— 3 843 016
US—A— 4 018 224

(73) Patentinhaber : Sterimed Gesellschaft für medizinischen Bedarf mbH
Fasanerieweg 13-17 Postfach 215
D-6600 Saarbrücken 3 (DE)

(72) Erfinder : WEJNAR, Siegfried
Strahlsunder Strasse 13
D-2120 Lüneburg (DE)

(74) Vertreter : Suchy, Herbert et al
Byk Gulden Lomberg Chemische Fabrik GmbH Patentabteilung Byk-Gulden-Strasse 2
D-7750 Konstanz (DE)

## Beschreibung

### Technisches Gebiet

Die Erfindung bezieht sich auf eine Unterdruckflasche für die Redon-Wunddrainage.

### Stand der Technik

Die postoperative Wunddrainage mittels hohem Unterdruck (Redon-Technik) hat eine sehr weite Verbreitung gefunden. In der DE-AS-26 39 715 wird eine einstückige Flasche aus Kunststoff zum einmaligen Gebrauch mit einem Druckanzeiger und einem Anschlußstutzen für einen zu einem Wunddrain führenden Schlauch beschrieben. Diese Flaschen werden unter sterilen Bedingungen mit einem Unterdruck von mehr als 90 kPa versehen. Beim Anlegen an einen Wunddrain kommt es daher zwischen der Wundhöhle, in der der Drain liegt, und der Umgebung zu einer entsprechenden Druckdifferenz. Durch diese Druckdifferenz werden die Wundflächen aneinander gepreßt, was zu einer Ruhigstellung des Wundspaltes führt. Die gewebliche Oberbrückung des Wundspaltes wird somit erleichtert, d. h. die Heilung der Wunde gefördert.

Bei der Wunddrainage nach Redon liegt ein geschlossenes System vor. Eine Kontamination über das Drainagesystem ist daher weitgehend ausgeschlossen. Kritisch ist jedoch eine Öffnung des Systems, was z. B. beim Wechseln der Saugflasche erforderlich ist. Besonders gefährlich ist ein Flaschenwechsel, bei dem versehentlich die Saugleitung vorher nicht dicht verschlossen wurde. In diesem Fall wird Sekret und keimbeladene Umgebungsluft in die Wunde gesaugt. Zudem wird die Druckdifferenz zwischen Umgebung und dem Inneren der Wunde ausgeglichen, was zu einer unerwünschten Aufhebung des Zusammenpressens der Wundränder führt. Bakteriologische Untersuchungen (A. Härle, Hygiene + Medizin 7[1982]427) ergeben, daß insbesondere der Flaschenwechsel die Sterilitätssituation des Wunddrainage-Systems negativ beeinflußt. Der Flaschenwechsel ist auch für das Pflegepersonal nicht unbedenklich, da die Gefahr der Berührung mit eventuell infektiösem Wundsekret besteht.

In manchen Anwendungsfällen ist es zweckmäßig, zu Beginn der Wunddrainage mit einem geringeren Unterdruck zu arbeiten. Dies kann bewerkstelligt werden, indem zuerst eine teilweise belüftete Unterdruckflasche eingesetzt wird, die dann später gegen eine Unterdruckflasche mit vollem Vakuum ausgetauscht wird. Auch hier kann es beim Flaschenwechsel zu Sterilitätsproblemen kommen.

### Beschreibung der Erfindung

Aufgabe der vorliegenden Erfindung war es, eine Unterdruckflasche für die Redon-Wunddrainage zur Verfügung zu stellen, mit der die mit dem Flaschenwechsel verbundenen Gefahren vermieden werden können.

Erfindungsgemäß gelöst wird diese Aufgabe dadurch, daß die Unterdruckflasche zwei voneinander getrennte Kammern umfaßt, die über ein T-Stück miteinander und mit einem Verbindungsschlauch in Verbindung stehen, wobei alle Zugänge des T-Stücks abklemmbar sind. Die beiden Kammern der erfindungsgegenständlichen Unterdruckflaschen weisen entweder ein im wesentlichen identisches Innenvolumen auf oder zeichnen sich durch unterschiedliche Innenvolumina aus.

Ein bevorzugter Gegenstand sind Unterdruckflaschen, deren beide Kammern jeweils mit einem Druckanzeiger ausgestattet sind.

Im Anlieferungszustand sind beide Kammern der erfindungsgemäßen Unterdruckflasche evakuiert und alle Anschlußstücke des T-Stücks mittels Klemmen abgeklemmt. Bei der Anwendung wird das freie Anschlußstück des T-Stücks mit dem zum Wunddrain führenden Verbindungsschlauch verbunden. Sodann werden die Klemme am ursprünglich freien Anschlußstück des T-Stücks und die Klemme zwischen T-Stück und der ersten Kammer der Unterdruckflasche geöffnet. Sobald bei herkömmlichen Einkammersaugflaschen ein Flaschenwechsel vorgenommen werden würde, wird die Klemme zwischen T-Stück und der ersten Kammer geschlossen und die Klemme zwischen T-Stück und der zweiten Kammer geöffnet. Weiteres Wundsekret fließt nun in die zweite Kammer. Dieses « Umschalten » von der ersten auf die zweite Kammer vermeidet die mit dem Flaschenwechsel verbundenen Risiken.

Gewünschtenfalls kann statt des beschriebenen « Umschaltens » das Schließen der Klemme zwischen T-Stück und erster Kammer unterlassen werden und durch Öffnen der Klemme zwischen T-Stück und zweiter Kammer ein Druckausgleich zwischen den beiden Kammern herbeigeführt werden. In diesem Fall steht für die weitere Drainage auch noch das restliche Volumen der ersten Kammer zur Verfügung.

Eine weitere Variante in der Anwendung der erfindungsgemäßen Doppelkammersaugflasche besteht darin, vor dem Herstellen einer leitenden Verbindung zwischen erster Kammer und Wunddrain, d. h. vor dem Öffnen der Klemme zwischen T-Stück und erster Kammer, den Unterdruck in der ersten Kammer auf einen gewünschten Wert zu reduzieren. Dies kann z. B. durch Anstechen eines als Druckanzeiger verwendeten Faltenbalgs oder eines anderen als selbstabdichtende Membran wirkenden Gummiteils mittels einer dünnen Punktionskanüle geschehen. Zur Vermeidung des Einschleppens von Keimen aus der Umluft ist die Kanüle an ihrem rückwärtigen Ende mit einem Bakterienfilter versehen. Durch diese Reduzierung des Drucks kann die Wunddrainage mit einem verringerten Unterdruck begonnen werden und später durch « Umschalten » auf die zweite Kammer oder « Zuschalten » der zweiten Kammer bei erhöhtem Unterdruck fortgesetzt werden. Diese Vorgangsweise wird bei bestimmten chirurgischen Operationen als vorteilhaft angesehen und kann mit der erfindungsgemäßen Doppelkammerflasche ohne Flaschenwechsel stattfinden.

Die erfindungsgemäße Unterdruckflasche wird durch an sich bekannte Verfahren hergestellt. Beispielsweise wird die Flasche aus zwei Halbflaschen z. B. durch Kleben oder Verschweißen zusammengefügt. Die Halbflaschen können auf übliche Weise im Blasverfahren hergestellt werden.

Das Volumen der Unterdruckflasche kann in weiten Grenzen je nach Verwendungszweck variiert werden. Zum Beispiel kann zur Drainage kleiner Wunden, wie sie z. B. bei handchirurgischen Operationen entstehen, eine Unterdruckflasche mit einem Gesamtvolumen von 100 bis 200 cm$^3$ bereitgestellt werden. Für größere Operationen können die Unterdruckflaschen ein Gesamtvolumen von beispielsweise 800 bis 1 000 cm$^3$ aufweisen. Bevorzugt sind Unterdruckflaschen mit einem Gesamtvolumen von 400 bis 600 cm$^3$.

Für besondere Zwecke können Unterdruckflaschen hergestellt werden, bei denen die beiden Kammern unterschiedliche Volumina besitzen.

Eine Ausgestaltung der Erfindung besteht darin, daß die Unterdruckflasche aus zwei getrennten Flaschen mit Druckanzeiger und Anschlußstutzen für eine Schlauchleitung besteht, die durch eine gemeinsame Halterung fest miteinander verbunden sind und deren Innenräume über ein T-Stück miteinander und mit einer Verbindungsleitung in Verbindung stehen.

Als Druckanzeiger kommen beispielsweise solche in Frage, wie sie bei den handelsüblichen Unterdruckflaschen für die Redon-Wunddrainage üblich sind. Weitere in Frage kommende Ausführungsformen für Druckanzeiger für Redon-Flaschen sind z. B. in der EP-A2-0 061 723 und der EP-A2-0 036 546 beschrieben.

Nachstehend wird die Erfindung anhand von zwei Ausführungsbeispielen näher erläutert.

Fig. 1 zeigt in teilweise schematisierter Darstellung eine Unterdruckflasche.

Fig. 2 zeigt eine weitere Ausführungsform.

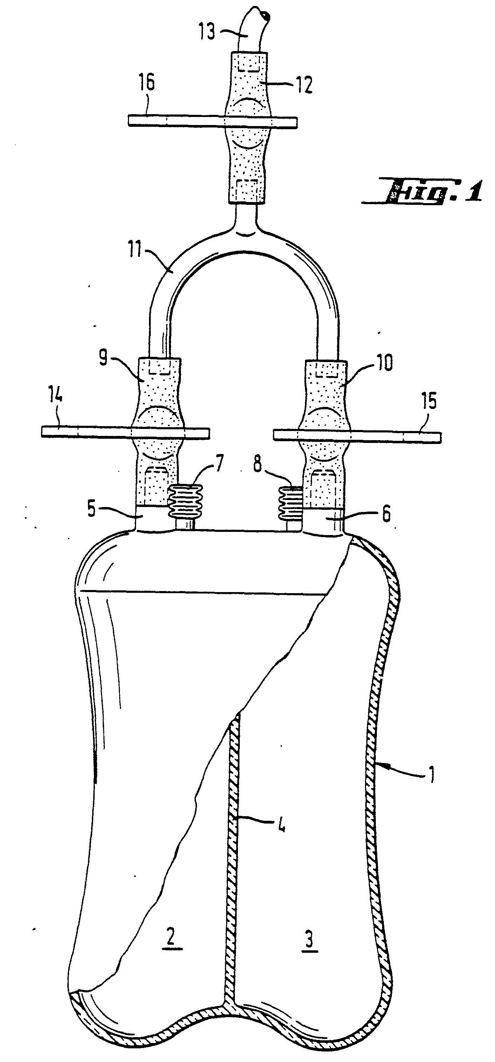

Die Unterdruckflasche 1 in Fig. 1 ist teilweise aufgebrochen gezeichnet. Eine Trennwand 4 trennt den Innenraum der Unterdruckflasche 1 in eine erste Kammer 2 und eine zweite Kammer 3. Die Unterdruckflasche 1 besitzt für jede der Kammern 2, 3 einen Anschlußstutzen 5, 6 und einen Druckanzeiger 7, 8. Die Druckanzeiger 7, 8 sind im Ausführungsbeispiel der Fig. 1 als Faltenbälge dargestellt. Auf die Anschlußstutzen 5, 6 sind Schlauchverbindungsstücke 9, 10 aufgezogen, die die Verbindung mit dem T-Stück 11 herstellen. Auf dem verbleibenden Ausgang des T-Stücks 11 ist ein Schlauchverbindungsstück 12 aufgesteckt, das die Verbindung zu dem zum Wunddrain (nicht dargestellt) führenden Verbindungsschlauch 13 herstellt. Auf den Schlauchverbindungsstücken 9, 10, 12 sind Schiebeklemmen 14, 15, 16 aufgesteckt.

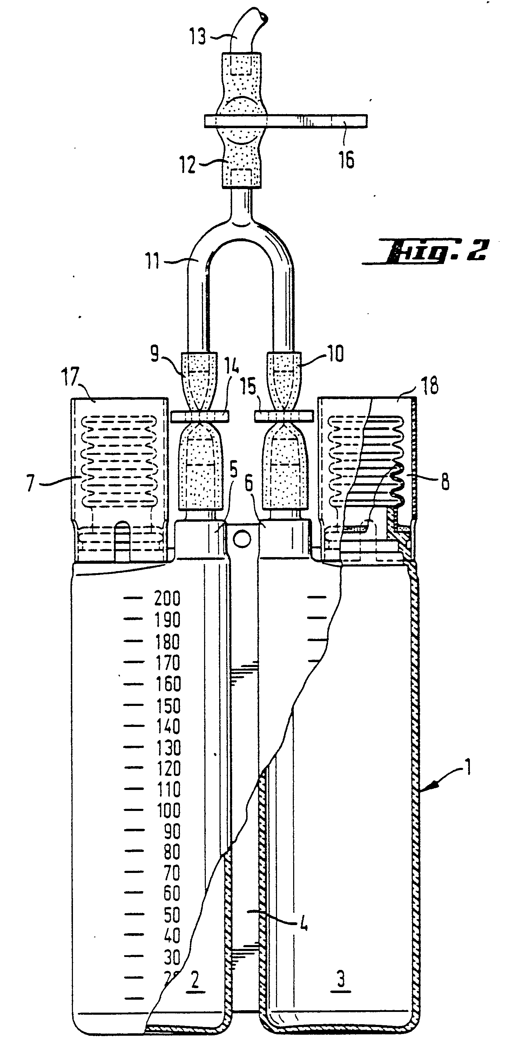

Fig. 2 zeigt eine weitere Ausführungsform einer Unterdruckflasche 1 in teilweise aufgebrochener Darstellung. Die Trennwand 4 ist hier als Verbindungssteg zwischen den beiden im Querschnitt im wesentlichen zylinderförmig ausgebildeten Kammern 2 und 3 gestaltet. Auf den Außenseiten der Kammern 2 und 3 sind Skalen aufgetragen anhand derer der jeweilige Füllungsgrad der Kammern abzulesen ist. Die Druckanzeiger 7 und 8 sind wie bei der Ausführungsform nach der Fig. 1 als Faltenbälge ausgestaltet, die von Hülsen 17 und 18 umgeben sind, auf denen Skalen für die Anzeige des Druckes in den Kammern 2 und 3 aufgebracht sein können. Die übrigen mit Bezugszeichen versehenen Elemente entsprechen denen in Fig. 1.

## Patentansprüche

1. Unterdruckflasche für die Redon-Wunddrainage, dadurch gekennzeichnet, daß die Unterdruckflasche (1) zwei voneinander getrennte Kammern (2, 3) umfaßt, die über ein T-Stück (11) miteinander und mit einem Verbindungsschlauch (13) in Verbindung stehen, wobei alle drei Zugänge des T-Stücks (11) abklemmbar sind.

2. Unterdruckflasche nach Anspruch 1, dadurch gekennzeichnet, daß die beiden Kammern (2, 3) ein im wesentlichen identisches Innenvolumen haben.

3. Unterdruckflasche nach Anspruch 1, dadurch gekennzeichnet, daß die beiden Kammern (2, 3) unterschiedliche Innenvolumina aufweisen.

4. Unterdruckflasche nach Anspruch 1, dadurch gekennzeichnet, daß die Kammern (2, 3) zusammen ein Innenvolumen von 100 bis 1 000, vorzugsweise 400 bis 600 cm$^3$ aufweisen.

5. Unterdruckflasche nach Anspruch 1, dadurch gekennzeichnet, daß die Kammern (2, 3) jede für sich als selbständige Unterdruckflasche ausgebildet ist, die durch eine gemeinsame Halterung fest miteinander verbunden sind.

6. Unterdruckflasche nach Anspruch 1, dadurch gekennzeichnet, daß die Kammern (2, 3) jeweils einen Druckanzeiger (7, 8) aufweisen.

## Claims

1. Suction bottle for Redon wound drainage, wherein the suction bottle (1) comprises two chambers (2, 3) separated from one another, which are connected to one another and to a connecting tube (13) via a T-piece (11), and all three inlets of the T-piece (11) can be pinched off.

2. Suction bottle as claimed in claim 1, wherein the two chambers (2, 3) have an essentially identical inner volume.

3. Suction bottle as claimed in claim 1, wherein the two chambers (2, 3) have different inner volumes.

4. Suction bottle as claimed in claim 2, wherein the chambers (2, 3) together have an inner volume of 100 to 1 000, preferably 400 to 600 cm$^3$.

5. Suction bottle as claimed in claim 1, wherein the chambers (2, 3) are each designed separately as independent suction bottles which are connected firmly to one another by means of a common mounting.

6. Suction bottle as claimed in claim 1, wherein the chambers (2, 3) each have a pressure indicator (7, 8).

## Revendications

1. Flacon à dépression pour drainage de blessures selon la technique Redon, caractérisé en ce que le flacon à dépression (1) comprend deux chambres (2, 3) séparées l'une de l'autre qui communiquent entre elles par un raccord en T (11) et avec un tuyau de liaison (13), les trois accès du raccord en T (11) pouvant être fermés par pincement.

2. Flacon à dépression selon la revendication 1, caractérisé en ce que les deux chambres (2, 3) ont un volume intérieur sensiblement identique.

3. Flacon à dépression selon la revendication 1, caractérisé en ce que les deux chambres (2, 3) présentent des volumes intérieurs différents.

4. Flacon à dépression selon la revendication 1, caractérisé en ce que les chambres (2, 3) présentent ensemble un volume intérieur de 100 à 1 000, de préférence de 400 à 600 cm$^3$.

5. Flacon à dépression selon la revendication 1, caractérisé en ce que les chambres (2, 3) sont réalisées chacune en soi sous forme d'un flacon à dépression autonome, lesquels sont reliés rigidement entre eux par une fixation commune.

6. Flacon à dépression selon la revendication 1, caractérisé en ce que les chambres (2, 3) comportent chacune un indicateur de pression (7, 8).

Fig. 1

Fig. 2